(19) Europäisches Patentamt
European Patent Office
Office européen des brevets

(11) Veröffentlichungsnummer: **0 322 707 A2**

(12) # EUROPÄISCHE PATENTANMELDUNG

(21) Anmeldenummer: **88121271.6**

(22) Anmeldetag: **20.12.88**

(51) Int. Cl.4: **C07C 103/30 , C07C 103/46 , C07C 153/01 , C07C 83/10 , C07C 103/34 , C07C 103/127 , A01N 37/38 , A01N 37/36 , C07C 69/66**

Patentanspr}che f}r folgende Vertragsstaaten: GR + SP.

(30) Priorität: **24.12.87 DE 3744021**

(43) Veröffentlichungstag der Anmeldung: **05.07.89 Patentblatt 89/27**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schlegel, Günter, Dr.**
**Feldbergstrasse 18**
**D-6237 Liederbach(DE)**
Erfinder: **Bauer, Klaus, Dr.**
**Doorner Strasse 53D**
**D-6450 Hanau(DE)**
Erfinder: **Bieringer, Hermann, Dr.**
**Eichenweg 26**
**D-6239 Eppstein/Taunus(DE)**

(54) **3-Phenoxy-phenylessigsäurederivate, Verfahren zu ihrer Herstellung und ihre Verwendung als Herbizide.**

(57) Die Erfindung betrifft herbizide Verbindungen (I), sowie deren Salze und Stereoisomere

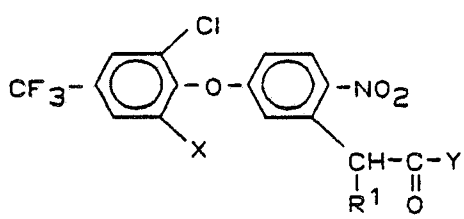

( I )

worin
X Halogen,
Y $NR^2R^3$ oder W-$(CR^2R^4)_n$-CO-Z-$R^5$,
W O oder S,
Z O, S oder $NR^1$,
n l, 2, 3 oder 4,
$R^1$,$R^4$ H oder niederes Alkyl, Alkoxy oder Halogenalkyl,
$R^2$,$R^3$ H oder gegebenenfalls substituiertes niederes Alkyl, Alkylaryl, Alkenyl, Alkinyl, Alkoxy oder Alkoxycarbonyl oder gegebenenfalls substituiertes Phenyl und
$R^5$ H, niederes Alkyl, Alkenyl oder Alkinyl oder $(C_5$-$C_7)$Cycloalkyl, gegebenenfalls substituiertes Phenyl oder für den Fall Z = $NR^1$ einen Rest $(C_1$-$C_4)$Alkoxy bedeutet.

EP 0 322 707 A2

Die Verbindungen (I) sind durch Nitrierung der Vorstufe von (I), in der die Nitrogruppe fehlt, erhältlich. Außerdem sind die Verbindungen (I) mit $R^1 = H$ durch Umsetzung der Carbonsäuren (Y = OH) oder deren reaktiven Ester oder Halogenide mit Verbindungen Y-H erhältlich.

Die herbiziden Verbindungen (I) sind gegen ein breites Spektrum schwerbekämpfbarer Unkräuter wirksam.

## 3-Phenoxy-phenylessigsäurederivate, Verfahren zu ihrer Herstellung und ihre Verwendung als Herbizide

Es ist bereits bekannt, daß substituierte Phenoxyphenylessigsäurederivate herbizide Eigenschaften aufweisen, siehe JP-A 6 2072-647 und EP-A 161 529. Diese weisen jedoch Nachteile bei der Anwendung auf, wie z. B. eine nicht ausreichende Selektivität oder zu geringe Wirksamkeit gegen wichtige schwerbekämpfbare Schadpflanzen.

Es wurden nun neue Phenoxyphenylessigsäurederivate gefunden, die überraschenderweise eine vorteilhaft selektive Herbizidwirkung besitzen und gegen ein breites Spektrum schwerbekämpfbarer Unkräuter wirken.

Gegenstand der vorliegenden Erfindung sind daher Verbindungen der Formel I, deren Salze und Stereoisomeren

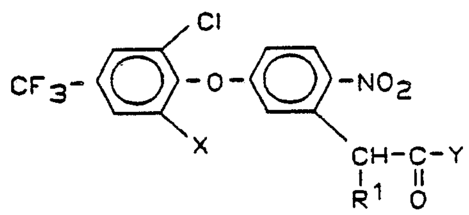

(I)

worin

X Halogen,

Y einen Rest der Formeln

$NR^2R^3$ oder

$W-(CR^2R^4)_n-C(=O)-Z-R^5$,

W O oder S,

Z O, S oder $NR^1$,

n 1, 2, 3 oder 4,

$R^1$,$R^4$ unabhängig voneinander Wasserstoff, $(C_1-C_4)$Alkyl, $(C_1-C_4)$Alkoxy oder Halogen$(C_1-C_4)$alkyl,

$R^2$,$R^3$ unabhängig voneinander Wasserstoff, $(C_1-C_4)$Alkyl, das ein- oder mehrfach durch Halogen und/oder ein-bis zweifach durch Cyano, $(C_1-C_4)$Alkoxy, $(C_1-C_4)$Alkylthio, Di$(C_1-C_4)$alkylamino, Carboxy, $(C_1-C_4)$Alkoxycarbonyl, Aminocarbonyl, $(C_1-C_4)$Alkylaminocarbonyl, Di$(C_1-C_4)$alkylaminocarbonyl, Tri$(C_1-C_4)$-alkylsilyl, unsubstituiertes oder durch $(C_1-C_4)$Alkyl, $(C_1-C_4)$Alkoxy oder Halogen substituiertes Phenyl, Furanyl oder Thienyl substituiert sein kann,
$(C_2-C_6)$Alkenyl, $(C_2-C_6)$Alkinyl, $(C_1-C_4$-Alkoxy)carbonyl, Phenyl, das durch $(C_1-C_4)$Alkyl, Halogen, Cyano oder $(C_1-C_4)$Alkoxy substituiert sein kann, oder $(C_1-C_4)$Alkoxy und

$R^5$ Wasserstoff, $(C_1-C_6)$Alkyl, das ein- oder mehrfach durch Halogen und/oder ein- bis zweifach durch Nitro, Cyano, $(C_1-C_4)$Alkoxy, $(C_1-C_4)$Alkylthio, Tri$(C_1-C_4)$alkylsilyl, unsubstituiertes oder durch $(C_1-C_4)$Alkyl, $(C_1-C_4)$Alkoxy oder Halogen substituiertes Phenyl, Furanyl oder Thienyl substituiert sein kann, ferner $(C_2-C_6)$Alkenyl, $(C_2-C_6)$Alkinyl, $(C_5-C_7)$Cycloalkyl, Phenyl, das durch $(C_1-C_4)$Alkyl, $(C_1-C_4)$Alkoxy, Halogen oder Cyano substituiert sein kann, oder für den Fall Z = $NR^1$ einen Rest $(C_1-C_4)$Alkoxy

bedeutet.

Bevorzugt von den Verbindungen der Formel I sind solche, bei denen

y $-W-(CR^2R^4)_n-C(=O)-Z-R^5$,

W O oder S,

Z O, S oder $NR^1$,

n 1 oder 2,

$R^1$ H oder $CH_3$,

$R^2$ H oder $(C_1-C_4)$Alkyl,

$R^4$ H, $(C_1-C_4)$Alkyl, das durch Halogen substituiert sein kann, $(C_1-C_4)$Alkoxy oder Benzyl bedeutet und

$R^5$ die obengenannten Bedeutungen besitzt.

Alkyl kann in obigen Definitionen geradkettig oder verzweigt sein. Halogen bedeutet insbesondere Fluor, Chlor oder Brom.

Im Falle $R^5$ = Wasserstoff oder $R^2$ oder $R^3$ = durch Carboxy substituiertes Alkyl können die Verbindungen der Formel I als Salze vorliegen, wobei solche Salze, die für die Landwirtschaft anwendbar sind, zum Einsatz kommen.

Als landwirtschaftlich einsetzbare Salze der Verbindungen der Formel I kommen beispielsweise in Frage: Na-, K-, Mg-, Ca-, Zn-Salze oder Ammonium- oder organische mono- bis tetra-substituierte Ammonium-, Sulfonium-, Sulfoxonium- oder Phosphoniumsalze, wobei als organische Reste übliche Substituenten wie beispielsweise Alkyl-, Hydroxyalkyly-, Cycloalkyl- oder gegebenenfalls substituierte Phenylreste in Frage kommen.

Die Verbindungen der Formel I besitzen in den Gruppierungen

$-CHR^1-\overset{O}{\overset{\parallel}{C}}-Y$ für den Fall $R^1 \neq H$ und

$W-(CR^2R^4)_n-\overset{O}{\overset{\parallel}{C}}-ZR^5$ für den Fall $R^2 \neq R^4$ und gegebenenfalls in den Resten $R^1$-$R^5$ selbst optisch aktive Zentren. Sie können daher als Stereoisomerengemische oder in Form der reinen Stereoisomeren vorliegen. Diese werden ebenfalls von der vorliegenden Erfindung umfaßt.

Gegenstand der vorliegenden Erfindung sind auch Verfahren zur Herstellung der Verbindungen der Formel I und deren Salze, dadurch gekennzeichnet, daß man a) eine Verbindung der Formel II

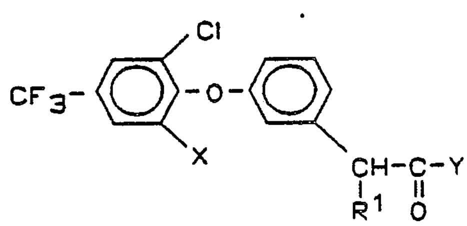

(II)

durch Umsetzung mit einem Nitrierungsmittel, bevorzugt Salpetersäure, Stickoxide oder Nitrate, umsetzt oder b) für $R^1$ = H eine Verbindung der Formel III

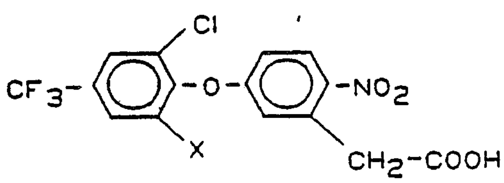

(III)

oder deren Ester oder Säurehalogenid mit einer Verbindung der Formel Y-H umsetzt und die erhaltenen Verbindungen gegebenenfalls in ihre Salze überführt.

Das Verfahren (a) kann wahlweise in Gegenwart von Schwefelsäure, Acetanhydrid, Eisessig oder einem Cosolvens* wie Dichlormethan gegebenenfalls unter Zugabe eines Katalysators wie Bortrifluoridetherat, Trifluoressigsäure, Methansulfonsäure, Trifluoressigsäureanhydrid oder eines Metallsalzes wie z. B. Thallium (I), Kupfer (I) oder Kupfer (II) durchgeführt werden.

Im Falle der Variante (b) können die Säurederivate wie Ester und Säurechloride anstelle von den an sich bekannten Verbindungen der Formel III (JP-A 6 2072-647) eingesetzt werden. Als Ester kommen insbesondere Alkylester wie Methyl- oder Ethylester in Betracht; als Säurehalogenide können insbesondere Säurechloride eingesetzt werden. Die Herstellung der Ester und Säurehalogenide erfolgt nach üblichen Methoden. Die Durchführung des Verfahrens (b) erfolgt nach an sich bekannten Methoden (s. z. B. Houben-Weyl, Bd. VIII, S. 503 ff. und S. 647 ff. (1952)).

Die Verbindungen der Formel II lassen sich aus den Verbindungen der Formel IV

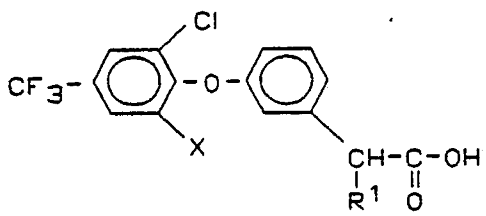

( IV )

oder deren Säurederivaten wie Estern oder Halogeniden nach den für Verfahren (b) genannten Methoden herstellen.

Die Verbindungen der Formel IV sind für $R^1$ = H bekannt (JP-A 6 2072-647). Die anderen Verbindungen der Formel IV werden auf analoge Weise hergestellt.

Geht man bei der Darstellung der Verbindungen I oder II von einem Säurechlorid aus, so kann man das bei der Umsetzung freiwerdende HCl-Gas durch einen Inertgasstrom oder durch Binden mit einem tert. Amin, Metallhydroxid oder Metallcarbonat entfernen.

Die erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Beispiele für solche Schadpflanzen sind verschiedene Arten von Avena, Lolium, Alopecurus, Phalaris, Echinochloa, Digitaria, Setaria, Cyperus, Agropyron, Cynodon, Imperata, Sorghum, Galium, Viola, Veronica, Lamium, Stellaria, Amaranthus, Sinapis, Ipomoea, Matricaria, Abutilon, Sida, Convolvulus, Cirsium, Rumex, Artemisia, Sagittaria, Alisma, Eleocharis und Scirpus. Dabei ist es gleichgültig, ob die Substanzen im Vorsaat-, Vorauflauf- oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrüber, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll-oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

## Formulierungsbeispiele

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz aus Inerstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (Triton x 207), 3 Gewichtsteilen Isotridecanolpolyglykolether (8 AeO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

## Chemische Beispiele

Beispiel I

N-(2-Methoxyethyl)-5-(2,6-dichlor-4-trifluormethylphenoxy)-2-nitro-phenylacetamid

4,10 g (0,01 mol) 5-(2,6-Dichlor-4-trifluormethylphenoxy)-2-nitro-phenylessigsäure werden in 50 ml Thionylchlorid 2 Stunden unter Rückfluß erhitzt und nach Abdestillieren des überschüssigen Thionylchlorids im Vakuum mit einer Mischung aus 0,75 g (0,01 mol) 2-Methoxyethylamin und 1,01 g (0,01 mol) Triethylamin in 60 ml Tetrahydrofuran (THF) versetzt. Nach einstündigem Rühren bei Raumtemperatur wird auf Eiswasser gegeben, mit Diethylether extrahiert, der Etherextrakt mit gesättigter $NaHCO_3$-Lösung (20 ml) und mit Wasser (50 ml) gewaschen, über $Na_2SO_4$ getrocknet und im Vakuum eingeengt. Es bleiben 4,12 g (88 % d. Th.) N-(2-methoxy-ethyl)-5-(2,6-dichlor-4-trifluormethylphenoxy)-2-nitro-phenylacetamid als weißer Feststoff vom Fp. 120 - 122 °C zurück.

Beispiel 2

1-Ethoxycarbonyl-ethyl-5-(2,6-dichlor-4-trifluormethylphenoxy)-2-nitro-phenylacetat

4,10 g (0,01 mol) 5-(2,6-Dichlor-4-trifluormethylphenoxy)-2-nitro-phenylessigsäure werden in 50 ml Dioxan gelöst und mit 1,62 g (0,01 mol) Carbonyldiimidazol versetzt. Nach Beendigung der Gasentwicklung wird noch 1/2 Stunden bei Raumtemperatur nachgerührt und mit 1,18 g (0,01 mol) Milchsäureethylester versetzt, 6 Stunden bei Raumtemperatur gerührt, auf Eiswasser gegeben und mit Diethylether extrahiert. Waschen der Etherphase mit gesättigter $NaHCO_3$-Lösung und Wasser, Trocknen über $Na_2SO_4$ und Einengen im Vakuum ergibt 4,29 g (84 %) 1-Ethoxycarbonylethyl-5-(2,6-dichlor-4-trifluormethylphenoxy)-2-nitro-phenylacetat als bernsteinfarbenes Wachs.

Auf analoge Weise lassen sich folgende Verbindungen der Formel I herstellen, siehe Tabelle 1.

**Tabelle 1**

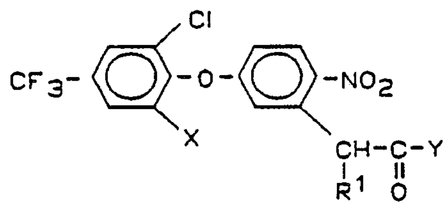

| Verb.-Nr. | X | $R^1$ | Y | Fp [°C] |
|---|---|---|---|---|
| 3 | Cl | H | $NHCH_3$ | 194/96 |
| 4 | Cl | H | $NH(CH_2)_3CH_3$ | 141/44 |
| 5 | Cl | H | $NHCH(CH_3)COOC_2H_5$ | 113/15 |
| 6 | Cl | H | $NHCH_2-C_6H_5$ | 183/85 |
| 7 | Cl | H | $NH-C_6H_4-2-Cl$ | |
| 8 | Cl | H | $NHCH_2CH_2CONH_2$ | |
| 9 | Cl | H | $N(CH_3)CH_2CH_2Si(CH_3)_3$ | |
| 10 | F | H | $CH_3$<br>$N(CH_3)-(CH_2)_2-Si-CH_3$<br>$CH_3$ | |
| 11 | Cl | H | | |
| 12 | Cl | H | $N(CH_3)OCH_3$ | 39/41 |
| 13 | Cl | H | $OCH_2COOCH_3$ | Wachs |
| 14 | Cl | H | $OCH_2COOCH(CH_3)_2$ | Wachs |
| 15 | Cl | H | $OCH_2COOCH_2CH_2Cl$ | Wachs |
| 16 | Cl | H | $OCH_2COOCH_2-C_6H_5$ | |
| 17 | Cl | H | $OCH(CH_3)COOH$ | |
| 18 | Cl | H | $O-CHCOOH$<br>$CH_2COOH$ | |
| 19 | Cl | H | $O-CH(CH_3)COONa$ | |

**Tabelle 1 (Fortsetzung)**

| Verb.-Nr. | X | $R^1$ | Y | Fp [°C] |
|---|---|---|---|---|
| 20 | Cl | H | $OCH(CH_3)COOK$ | |
| 21 | Cl | H | $O(CH_2)_2COOH$ | |
| 22 | Cl | H | $OCH(CH_3)CONHCH(CH_3)_2$ | |
| 23 | Cl | H | $O(CH_2)_2CONH_2$ | |
| 24 | Cl | H | $OCH(C_2H_5)COOC_2H_5$ | |
| 25 | Cl | H | $OCH(C_2H_5)COOH$ | |
| 26 | Cl | H | $OCH(CH_3)CONHn\text{-}C_6H_{13}$ | |
| 27 | Cl | H | $O(CH_2)_2CONHCH_2$- 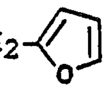 | |
| 28 | Cl | H | $OCH(CH_3)CONH\text{-}CH_2\text{-}C_6H_4\text{-}2\text{-}Cl$ | |
| 29 | Cl | H | $OCH(CH_3)CH_2CONH\text{-}C_6H_5$ | |
| 30 | Cl | H | $OCH(CH_3)COO\text{-}C_6H_4\text{-}4\text{-}Cl$ | |
| 31 | Cl | H | $OCH(CH_3)CONH\text{-}C_6H_3\text{-}2,4\text{-}Cl_2$ | |
| 32 | Cl | H | $OCH(CH_3)CONH\text{-}C_6H_4\text{-}4\text{-}OCH_3$ | |
| 33 | Cl | H | $O(CH_2)_2CONH(CH_2)_2CN$ | |
| 34 | Cl | H | $SCH_2COOC_2H_5$ | Wachs |
| 35 | Cl | H | $SCH(CH_3)COOCH_3$ | Wachs |
| 36 | Cl | H | $S(CH_2)_2COOC_2H_5$ | |
| 37 | Cl | H | $SCH_2COOCH_2$- 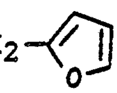 | |
| 38 | Cl | H | $SCH(CH_3)CONHCH_2\text{-}C_6H_5$ | |

**Tabelle 1** (Fortsetzung)

| Verb.-Nr. | X | $R^1$ | Y | Fp [°C] |
|---|---|---|---|---|
| 39 | Cl | $CH_3$ | $NHCH(CH_3)_2$ | |
| 40 | Cl | $CH_3$ | $NH(CH_2)_2OCH_3$ | |
| 41 | Cl | $CH_3$ | $OCH(CH_3)COOCa/2$ | |
| 42 | Cl | $CH_3$ | $OCH(CH_3)CONH(CH_2)_2CH_3$ | |
| 43 | Cl | $CH_3$ | $SCH(CH_3)COOCH_2Si(CH_3)_3$ | |
| 44 | Cl | $CH_3$ | $S(CH_2)_2COOCH_2C{\equiv}CH$ | |
| 45 | Cl | $OCH_3$ | $OCH_2COOCH_2CH_2Cl$ | |
| 46 | Cl | $OCH_3$ | $OCH_2\overset{O}{\overset{\Vert}{C}}SCH_3$ | |
| 47 | Cl | $OCH_3$ | $SCH(CH_3)\overset{O}{\overset{\Vert}{C}}SC_2H_5$ | |
| 48 | F | H | $NHCH_2CH_2CN$ | |
| 49 | F | H | $NHCH_2CH{=}CH_2$ | |
| 50 | F | H | $NHCH(CH_3)CON(CH_3)_2$ | |
| 51 | F | H | $NHCOOC_2H_5$ | |
| 52 | F | H | $NH-C_6H_4-4-OCH_3$ | |
| 53 | F | H | $NHCH_2-C_6H_4-4-CH_3$ | |
| 54 | F | H | $OCH_2COOC_2H_5$ | Wachs |
| 55 | F | H | $OCH(CH_3)COOCH_3$ | Wachs |
| 56 | F | H | $OCH(CH_3)COOC_2H_5$ | Wachs |
| 57 | F | H | $OCH_2COOCH_2-C_6H_5$ | |
| 58 | F | H | $OCH_2COOCH_2-$ 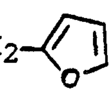 | |
| 59 | F | H | $OCH_2COO(CH_2)_2-C_6H_4-4-Cl$ | |
| 60 | F | H | $OCH-COOC_2H_5$<br>$CH_2-COOC_2H_5$ | Wachs |

**Tabelle 1** (Fortsetzung)

| Verb.-Nr. | X | $R^1$ | Y | Fp [°C] |
|---|---|---|---|---|
| 61 | F | H | $OCHCOOC_2H_5$<br>$C_6H_5$ | |
| 62 | F | H | $OCH(CH_3)CONH(CH_2)_3CH_3$ | |
| 63 | F | H | $OCH(CH_3)CONH(CH_2)_2OCH_3$ | |
| 64 | F | H | $OCH(CH_3)CONH-$ 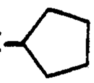 | |
| 65 | F | H | $OCH_2CH_2COO$-Cyclohexyl | |
| 66 | F | H | $OCH(CH_3)CONHCH_2COOC_2H_5$ | |
| 67 | F | H | $OCH(CH_3)CONHCH_2-C_6H_5$ | |
| 68 | F | $CH_3$ | $NHCH_2-$ 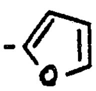 | |
| 69 | F | $CH_3$ | $OCH(CH_3)COOCH_3$ | |
| 70 | F | $CH_3$ | $OC(CH_3)_2COOC_2H_5$<br>$COOC_2H_5$ | |
| 71 | F | $CH_3$ | $O(CH_2)_2CONHCHCH_3$ | |
| 72 | F | $C_2H_5$ | $OCH(CH_3)CON(C_2H_5)_2$ | |
| 73 | F | $C_2H_5$ | $OCH_2COOC_2H_5$ | |
| 74 | F | $C_2H_5$ | $NH(CH_2)_3SCH_3$ | |
| 75 | F | $CH_2Cl$ | $NH(CH_2)_2N(CH_3)_2$ | |
| 76 | F | $CCl_3$ | $OCH_2COOCH_3$ | |
| 77 | F | $OCH_3$ | $NH_2$ | |
| 78 | F | $OCH_3$ | $OCH_2COOCH_3$ | |
| 79 | F | $OCH_3$ | $OCH_2CONHC_2H_5$ | |

**Tabelle 1 (Fortsetzung)**

| Verb.-Nr. | X | $R^1$ | Y | Fp [°C] |
|---|---|---|---|---|
| 80 | Cl | H | $S(CH_2)_2COOCH_3$ | Wachs |
| 81 | Cl | H | 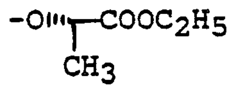 | Wachs; $[\alpha]_D^{25}= -16,1°$ |
| 82 | Cl | H | $NH_2$ | 184/86 |
| 83 | Cl | H | $OC(CH_3)_2COOCH_3$ | Wachs |
| 84 | Cl | H | $O(CH_2)COOC_2H_5$ | Wachs |
| 85 | F | H | $NH(CH_2)_2OCH_3$ | 134/36 |
| 86 | F | H | $SCH_2COOC_2H_5$ | Wachs |
| 87 | Cl | H | $OCH(CH_3)CON(C_2H_5)_2$ | Wachs |
| 88 | Cl | H | $OCH(C_6H_5)COOC_2H_5$ | 145/47 |
| 89 | Cl | H | $OCH(CH_3)CONHCH_2C_6H_5$ | 129/31 |
| 90 | Cl | H | 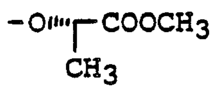 | Wachs; $[\alpha]_D^{25}=-18,95°$ |
| 91 | Cl | H | 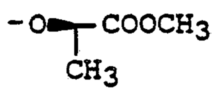 | Wachs; $[\alpha]_D^{25}=+18,0°$ |
| 92 | Cl | H | $OCH(CH_3)CONH_2$ | 78/80 |
| 93 | Cl | H | $OCH(C_2H_5)CONHCH_2C_6H_5$ | 129/31 |
| 94 | Cl | H | $OCH(CH_3)CH_2COOC_2H_5$ | Wachs |
| 95 | Cl | H | $OCH(CH_3)COOCH(CH_3)_2$ | Wachs |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

**1. Unkrautwirkung im Vorauflauf**

Samen bzw. Rhizomstücke von mono- und dikotylen Unkrautpflanzen wurden in Plastiktöpfen (Durchmesser: 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten (Temperatur: 23 plus/minus 2 Grad Celsius; relative Luftfeuchte: 60 - 80

%).

Die optische Bonitur der Pflanzen- bzw. der Auflaufschäden erfolgte nach dem Auflaufen der Versuchspflanzen nach einer Versuchszeit von 3 - 4 Wochen im Vergleich zu unbehandelten Kontrollen. Wie die Boniturwerte in Tabelle 1 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono- und dikotylen Unkräutern wurden in Plastiktöpfen (Durchmesser = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 - 800 l/ha auf die grünen Pflanzenteile gesprüht und nach ca. 3 - 4 Wochen Standzeit der Versuchspflanzen im Gewächshaus unter optimalen Wachstumsbedingungen (Temperatur: 23 plus/minus 2 Grad Celsisus, relative Luftfeuchte 60 - 80 %) die Wirkung der Präparate optisch im Vergleich zu unbehandelten Kontrollen bonitiert.

Die erfindungsgemäßen Mittel weisen auch im Nachauflauf eine gute herbizide Wirksamkeit gegne ein breites Spektrum wirtschaftlich wichtiger Ungräser und Unkräuter auf (Tabelle 2).

**3. Kulturpflanzenverträglichkeit**

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1 beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweiblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die erfindungsgemäßen Verbindungen weisen somit eine hohe Selektivität bei Anwendung zur Bekämpfung von unerwünschtem Pflanzenwuchs in landwirtschaftlichen Kulturen auf.

**Tabelle 2:** Vorauflaufwirkung

| Beispiel-Nr. | Dosis | herbizide Wirkung | | | | |
|---|---|---|---|---|---|---|
| | kg a.i./ha | SIA | CRS | STM | LOM | ECG |
| 1 | 2,5 | 5 | 5 | 5 | 5 | 5 |
| 2 | 2,5 | 5 | 5 | 5 | 3 | 4 |
| 3 | 2,5 | 2 | 4 | 5 | 2 | 4 |
| 4 | 2,5 | 2 | 4 | 5 | 3 | 3 |
| 5 | 1,25 | 5 | 5 | 5 | 5 | 4 |
| 6 | 1,25 | 5 | 5 | 4 | 2 | 5 |
| 12 | 2,5 | 5 | 5 | 5 | 5 | 5 |
| 13 | 1,25 | 5 | 2 | 5 | 2 | 2 |
| 34 | 1,25 | 5 | 5 | 5 | 4 | 5 |
| 56 | 1,25 | 5 | 5 | 5 | 5 | 5 |
| 80 | 1,25 | 5 | 5 | 5 | 3 | 5 |
| 81 | 1,25 | 5 | 4 | 5 | 3 | 3 |
| 82 | 1,25 | 5 | 5 | 5 | 4 | 5 |

**Fortsetzung Tabelle 2**

| Beispiel-Nr. | Dosis kg a.i./ha | herbizide Wirkung SIA | CRS | STM | LOM | ECG |
|---|---|---|---|---|---|---|
| 83 | 1,25 | 5 | 5 | 5 | 2 | 2 |
| 84 | 1,25 | 5 | 3 | 5 | 3 | 2 |
| 85 | 1,25 | 5 | 5 | 5 | 5 | 5 |
| 86 | 1,25 | 5 | 5 | 5 | 5 | 5 |
| 87 | 1,25 | 4 | 3 | 3 | 2 | 2 |
| 88 | 1,25 | 2 | 2 | 2 | 1 | 1 |
| 89 | 1,25 | 5 | 5 | 5 | 4 | 2 |
| 90 | 1,25 | 3 | 4 | 5 | 3 | 1 |
| 91 | 1,25 | 4 | 5 | 5 | 2 | 2 |
| 92 | 1,25 | 5 | 2 | 5 | 2 | 1 |
| 93 | 1,25 | 4 | 5 | 5 | 2 | 0 |

Abkürzungen:
SIA = Sinapis alba
CRS = Chrysanthemum segetum
STM = Stellaria media
LOM = Lolium multiflorum
ECG = Echinochloa crus-galli

**Beispiel 2: Nachauflaufwirkung**

| Beispiel-Nr. | Dosis kg a.i./ha | herbizide Wirkung SIA | CRS | STM | LOM | ECG |
|---|---|---|---|---|---|---|
| 1 | 2,5 | 5 | 5 | 5 | 5 | 5 |
| 2 | 2,5 | 5 | 5 | 5 | 4 | 4 |
| 3 | 2,5 | 5 | 5 | 4 | 2 | 2 |
| 4 | 2,5 | 4 | 4 | 4 | 2 | 1 |

**Beispiel 2: Nachauflaufwirkung**

| Beispiel-Nr. | Dosis kg a.i./ha | herbizide Wirkung SIA | CRS | STM | LOM | ECG |
|---|---|---|---|---|---|---|
| 5 | 1,25 | 5 | 5 | 5 | 5 | 5 |
| 6 | 1,25 | 3 | 4 | 5 | 4 | 3 |
| 12 | 2,5 | 5 | 5 | 5 | 2 | 2 |
| 13 | 1,25 | 5 | 5 | 5 | 4 | 4 |
| 34 | 1,25 | 5 | 5 | 5 | 3 | 5 |
| 56 | 1,25 | 5 | 5 | 5 | 5 | 5 |
| 80 | 1,25 | 5 | 5 | 5 | 3 | 5 |
| 81 | 1,25 | 5 | 5 | 5 | 3 | 3 |
| 82 | 1,25 | 3 | 4 | 4 | 2 | 4 |
| 83 | 1,25 | 5 | 5 | 5 | 2 | 3 |
| 84 | 1,25 | 5 | 5 | 5 | 3 | 4 |
| 85 | 1,25 | 5 | 5 | 5 | 5 | 5 |
| 86 | 1,25 | 5 | 5 | 5 | 5 | 5 |
| 87 | 1,25 | 5 | 5 | 4 | 2 | 4 |
| 88 | 1,25 | 5 | 5 | 3 | 2 | 3 |
| 89 | 1,25 | 5 | 5 | 4 | 2 | 3 |
| 90 | 1,25 | 5 | 5 | 5 | 1 | 1 |
| 91 | 1,25 | 5 | 5 | 5 | 1 | 2 |
| 92 | 1,25 | 5 | 5 | 5 | 4 | 4 |
| 93 | 1,25 | 4 | 3 | 3 | 1 | 2 |
| 94 | 1,25 | 5 | 5 | 5 | 2 | 3 |

## Ansprüche

1. Verbindungen der Formel I, deren Salze und Stereoisomere,

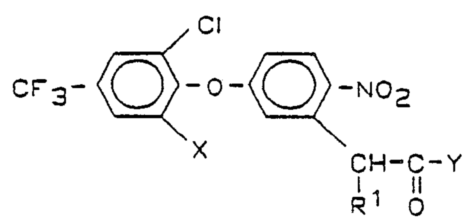

(I)

worin

X Halogen,

Y einen Rest der Formeln
$NR^2R^3$ oder
$W-(CR^2R^4)_n-\overset{O}{\overset{\|}{C}}-Z-R^5$,
W O oder S,
Z O, S oder $NR^1$,
n 1, 2, 3 oder 4,
$R^1$,$R^4$ unabhängig voneinander Wasserstoff, $(C_1-C_4)$Alkyl, $(C_1-C_4)$Alkoxy oder Halogen$(C_1-C_4)$alkyl,
$R^2$,$R^3$ unabhängig voneinander Wasserstoff, $(C_1-C_4)$Alkyl, das ein- oder mehrfach durch Halogen und/oder ein- bis zweifach durch Cyano, $(C_1-C_4)$Alkoxy, $(C_1-C_4)$Alkylthio, Di$(C_1-C_4)$alkylamino, Carboxy, $(C_1-C_4)$Alkoxycarbonyl, Aminocarbonyl, $(C_1-C_4)$Alkylaminocarbonyl, Di$(C_1-C_4)$alkylaminocarbonyl, Tri$(C_1-C_4)$alkylsilyl, unsubstituiertes oder durch $(C_1-C_4)$Alkyl, $(C_1-C_4)$Alkoxy oder Halogen substituiertes Phenyl, Furanyl oder Thienyl substituiert sein kann, $(C_2-C_6)$Alkenyl, $(C_2-C_6)$Alkinyl, $(C_1-C_4$-Alkoxy)carbonyl, Phenyl, das durch $(C_1-C_4)$Alkyl, Halogen, Cyano oder $(C_1-C_4)$Alkoxy substituiert sein kann, oder $(C_1-C_4)$Alkoxy und
$R^5$ Wasserstoff, $(C_1-C_6)$Alkyl, das ein- oder mehrfach durch Halogen und/oder ein- bis zweifach durch Nitro, Cyano, $(C_1-C_4)$Alkoxy, $(C_1-C_4)$Alkylthio, Tri$(C_1-C_4)$alkylsilyl, unsubstituiertes oder durch $(C_1-C_4)$Alkyl, $(C_1-C_4)$Alkoxy oder Halogen substituiertes Phenyl, Furanyl oder Thienyl substituiert sein kann, ferner $(C_2-C_6)$Alkenyl, $(C_2-C_6)$Alkinyl, $(C_5-C_7)$Cycloalkyl, Phenyl, das durch $(C_1-C_4)$Alkyl, $(C_1-C_4)$Alkoxy, Halogen oder Cyano substituiert sein kann, oder für den Fall Z = $NR^1$ einen Rest $(C_1-C_4)$Alkoxy
bedeutet,

2. Verbindungen der Formel I von Anspruch 1, worin
Y $-W-(CR_2R_4)_n-\overset{O}{\overset{\|}{C}}-Z-R_5$,
W O oder S,
Z O, S oder $NR^1$,
n 1 oder 2,
$R^1$ H oder $CH_3$,
$R^2$ H oder $(C_1-C_4)$Alkyl,
$R^4$ H, $(C_1-C_4)$Alkyl, das durch Halogen substituiert sein kann, Alkoxy oder Benzyl bedeutet und
$R^5$ die in Anspruch 1 genannte Bedeutung besitzt.

3. Verfahren zur Herstellung der Verbindungen der Formel I nach Anspruch 1, ihrer Isomeren und Salze, dadurch gekennzeichnet, daß man
a) eine Verbindung der Formel II

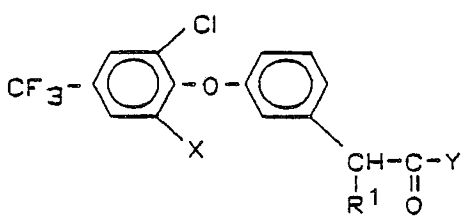

(II)

durch Umsetzung mit einem Nitrierungsmittel umsetzt, oder
b) für $R^1$ = H eine Verbindung der Formel III

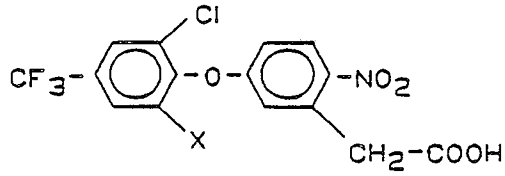

(III)

oder deren Ester oder Säurehalogenid mit einer Verbindung der Formel Y-H umsetzt und die erhaltenen Verbindungen gegebenenfalls in ihre Salze überführt.

4. Herbizide Mittel, dadurch gekennzeichnet, daß sie eine wirksame Menge einer Verbindung der Formel I von Ansprüchen 1 oder 2 oder deren Salze oder Stereoisomere enthalten.

5. Verwendung von Verbindungen der Formel I von Ansprüchen 1 oder 2 oder deren Salze oder Stereoisomeren als Herbizide.

6. Verfahren zur Bekämpfung von unerwünschten Pflanzen, dadurch gekennzeichnet, daß man auf diese oder die Nutzflächen eine wirksame Menge einer Verbindung der Formel I von Ansprüchen 1 oder 2 oder deren Salze oder Stereoisomeren appliziert.

Patentansprüche für folgenden Vertragsstaaten: ES, GR.

1. Verfahren zur Herstellung der Verbindungen der Formel I, ihrer Isomeren und Salze,

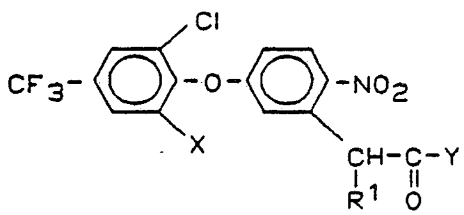

( I )

worin

X  Halogen,

Y  einen Rest der Formeln

$NR^2R^3$ oder

$W-(CR^2R^4)_n-\overset{O}{\overset{\|}{C}}-Z-R^5$,

W  O oder S,

Z  O, S oder $NR^1$,

n  1, 2, 3 oder 4,

$R^1,R^4$  unabhängig voneinander Wasserstoff, $(C_1-C_4)$Alkyl, $(C_1-C_4)$Alkoxy oder Halogen$(C_1-C_4)$alkyl,

$R^2,R^3$  unabhängig voneinander Wasserstoff, $(C_1-C_4)$Alkyl, das ein- oder mehrfach durch Halogen und/oder eir bis zweifach durch Cyano, $(C_1-C_4)$Alkoxy, $(C_1-C_4)$Alkylthio, Di$(C_1-C_4)$alkylamino, Carboxy, $(C_1-C_4)$Alkoxycarbonyl, Aminocarbonyl, $(C_1-C_4)$Alkylaminocarbonyl, Di$(C_1-C_4)$alkylaminocarbonyl, Tri$(C_1-C_4)$-alkylsilyl, unsubstituiertes oder durch $(C_1-C_4)$Alkyl, $(C_1-C_4)$Alkoxy oder Halogen substituiertes Phenyl, Furanyl oder Thienyl substituiert sein kann, $(C_2-C_6)$Alkenyl, $(C_2-C_6)$Alkinyl, $(C_1-C_4$-Alkoxy)carbonyl, Phenyl, das durch $(C_1-C_4)$Alkyl, Halogen, Cyano oder $(C_1-C_4)$Alkoxy substituiert sein kann, oder $(C_1-C_4)$Alkoxy und

$R^5$  Wasserstoff, $(C_1-C_6)$Alkyl, das ein- oder mehrfach durch Halogen und/oder ein- bis zweifach durch Nitro, Cyano, $(C_1-C_4)$Alkoxy, $(C_1-C_4)$Alkylthio, Tri$(C_1-C_4)$alkylsilyl, unsubstituiertes oder durch $(C_1-C_4)$Alkyl $(C_1-C_4)$Alkoxy oder Halogen substituiertes Phenyl, Furanyl oder Thienyl substituiert sein kann, ferner $(C_2-C_6)$Alkenyl, $(C_2-C_6)$Alkinyl, $(C_5-C_7)$Cycloalkyl, Phenyl, das durch $(C_1-C_4)$Alkyl. $(C_1-C_4)$Alkoxy, Halogen oder Cyano substituiert sein kann, oder für den Fall Z = $NR^1$ einen Rest $(C_1-C_4)$Alkoxy

bedeutet, dadurch gekennzeichnet, daß man

a) eine Verbindung der Formel II

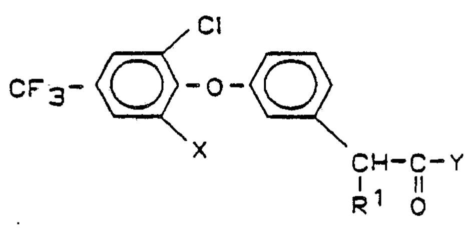

( II )

durch Umsetzung mit einem Nitrierungsmittel umsetzt, oder

b) für $R^1$ = H eine Verbindung der Formel III

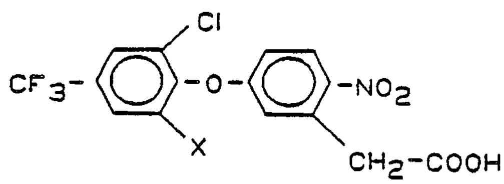

(III)

oder deren Ester oder Säurehalogenid mit einer Verbindung der Formel Y-H umsetzt und die erhaltenen Verbindungen gegebenenfalls in ihre Salze überführt.

2. Verfahren nach Anspruch l, worin

y $-W-(CR^2R^4)_n-\overset{O}{\overset{\|}{C}}-Z-R^5$,
W O oder S,
Z O, S oder $NR^1$,
n 1 oder 2,
$R^1$ H oder $CH_3$,
$R^2$ H oder $(C_1-C_4)$Alkyl,
$R^4$ H, $(C_1-C_4)$Alkyl, das durch Halogen substituiert sein kann, $(C_1-C_4)$Alkoxy oder Benzyl bedeutet und
$R^5$ die in Anspruch 1 genannte Bedeutung besitzt.

3. Verfahren nach Anspruch 1 oder 2, dadurch gekennzeichnet, daß nach a) mit einem Nitrierungsmittel aus der Gruppe Salpetersäure, Stickoxide und Nitrate und in Gegenwart von Schwefelsäure, Acetanhydrid, Eisessig oder einem Cosolvens wie Dichlormethan umgesetzt wird.

4. Verfahren nach Anspruch 3, dadurch gekennzeichnet, daß zusätzlich in Gegenwart eines Katalysators wie Bortrifluoridetherat, Trifluoressigsäure, Methansulfonsäure, Trifluoressigsäureanhydrid oder eines Metallsalzes aus der Reihe Thallium (I), Kupfer (I) und Kupfer (II) umgesetzt wird.

5. Verwendung der Verbindung der Formel I nach Anspruch 1 oder 2 oder deren Salze oder Stereoisomeren als Herbizide.

6. Verfahren zur Bekämpfung von unerwünschten Pflanzen, dadurch gekennzeichnet, daß man auf diese oder die Nutzflächen eine wirksame Menge einer Verbindung der Formel I nach Anspruch 1 oder 2 oder deren Salze oder Stereoisomeren appliziert.